# EUROPEAN PATENT SPECIFICATION

(11) **EP 2 806 853 B1**
(45) Date of publication and mention of the grant of the patent: **08.07.2020**
(21) Application number: 13702182.0
(22) Date of filing: 22.01.2013
(51) Int. Cl.: A61K 47/34, A61K 9/06, A61K 9/00, A61K 9/50

(54) **TIME RELEASED BIODEGRADABLE OR BIOERODIBLE MICROSPHERES OR MICROPARTICLES SUSPENDED IN A SOLIDIFYING DEPOT-FORMING INJECTABLE DRUG FORMULATION**
ZEITLICH FREIGEGEBENE, BIOLOGISCH ABBAUBARE ODER BIOLOGISCH ERODIERBARE MIKROKUGELN ODER MIKROPARTIKEL IN EINER SICH VERFESTIGENDEN, DEPOTBILDENDEN, INJIZIERBAREN ARZNEIMITTELFORMULIERUNG
MICROSPHÈRES BIOÉRODABLES OU BIODÉGRADABLES À LIBÉRATION PROLONGÉE OU MICROPARTICULES EN SUSPENSION DANS UNE FORMULATION D'UN MÉDICAMENT INJECTABLE À FORMATION DE DÉPÔT DE SOLIDIFICATION

(30) Priority: 23.01.2012 US 201261589681 P
(43) Date of publication of application: 03.12.2014
(73) Proprietor: ALLERGAN, INC., Irvine, CA 92612 (US)
(72) Inventor: MARSH, David, A., Irvine California 92618 (US); RIVERS, Hongwen Ma, San Diego California 92127 (US)
(74) Representative: Hoffmann Eitle
(86) International application number: PCT/US2013/022466
(87) International publication number: WO 2013/112434

(56) References cited:
- EP-A1- 1 568 359
- US-A- 5 441 732
- US-A- 5 744 153
- US-A1- 2002 076 441
- US-A1- 2007 202 186
- US-A1- 2011 104 151
- YOON KI JOUNG ET AL: "PLGA microparticle-embedded thermosensitive hydrogels for sustained release of hydrophobic drugs; PLGA microparticle-embedded thermosensitive hydrogels", BIOMEDICAL MATERIALS, INSTITUTE OF PHYSICS PUBLISHING, BRISTOL, GB, vol. 2, no. 4, 1 December 2007 (2007-12-01), pages 269-273, XP020132840, ISSN: 1748-605X

## Description

### BACKGROUND

There is a continuing need for methods of improved sustained delivery of drugs to human beings and animals.

US5441732 (A) discloses reversibly gelling aqueous and oil emulsions which can be modified to incorporate oil soluble pharmaceutical compounds for delivery to physiological systems in a soluble form and which undergo changes in viscosity in response to substantially simultaneous changes in both temperature and pH. The compositions are formed of relatively low concentrations of a stable combination of at least one pH-sensitive reversibly gelling polymer, at least one temperature-sensitive reversibly gelling polymer and at least one organic oil. According to this document, the compositions can be formulated to exhibit a sol-gel transition over a wide range of conditions and viscosities and may be modified to incorporate a pharmaceutical compound for utilization as droppable or injectable drug delivery systems which will gel following administration to a physiological system for the sustained delivery of such pharmaceutical compounds.

EP1568359 (A1) discloses a drug delivery system to a posterior segment wherein a pharmaceutical composition comprising a drug and a vehicle is administered subconjunctivally to form a depot out of the vehicle. The pharmaceutical composition comprises the vehicle in the form of gel and the drug suspended in the vehicle.

US5744153A discloses biocompatible liquid delivery compositions for the formation of sustained release delivery systems for active agents. The compositions include liquid formulations of a biocompatible polymer or prepolymer in combination with a controlled release component. The controlled release component includes an active agent. These compositions may be introduced into the body of a subject in liquid form which then solidify or cure in situ to form a controlled release implant or a film dressing.

US20020076441A1 discloses a composition and method for releasing a bioactive agent or a drug within a biological environment in a controlled manner. The composition is a dual phase polymeric agent-delivery composition comprising a continuous biocompatible gel phase, a discontinuous particulate phase comprising defined microparticles and an agent to be delivered. A microparticle containing a bioactive agent is releasably entrained within a biocompatible polymeric gel matrix. The bioactive agent may be contained in the microparticle phase alone or in both the microparticles and the gel matrix. The release of the agent is prolonged over a period of time, and the delivery may be modulated and/or controlled. In addition, a second agent may be loaded in some of the microparticles and/or the gel matrix.

US20070202186A1 provides drug formulations, devices and methods to deliver biologically active substances to the eye. The formulations are delivered into scleral tissues adjacent to or into the suprachoroidal space without damage to the underlying choroid. One class of formulations is provided wherein the formulation is localized in the suprachoroidal space near the region into which it is administered. Another class of formulations is provided wherein the formulation can migrate to another region of the suprachoroidal space.

US20110104151A1 discloses pharmaceutical compositions comprising microparticles that are useful for treating or preventing age-related macular degeneration. Also disclosed are microparticles that can be used to treat or prevent macular angiogenesis. Further disclosed are methods of making the microparticles and compositions and methods for treating or preventing macular degeneration and diseases, illnesses, or conditions relating to increased or abnormal macular angiogenesis.

Biomedical Materials 2(4):269-273 discloses PLGA microparticle-embedded thermosensitive hydrogels for sustained release of hydrophobic drugs.

### SUMMARY

An injectable liquid depot forming material may be used to implant a solid sustained drug delivery device in a human being or animal without the need for making an incision in the body. This may be useful for sensitive areas such as an eye, where incision may be risky. Since the material may be in a liquid form, the size of the device may not be limited by the diameter of a needle. For example, a solid implant may only be injected if it has a cross-sectional area that allows it to pass through a needle. Thus, a smaller needle size may be used for injectable liquid depot forming materials. It may also provide more flexible dosing than a solid implant, since the amount of depot material administered may be easily varied.

The invention is defined in the claims and relates to a composite drug delivery material comprising: a plurality of microparticles dispersed in a media composition. The microparticles comprise: a drug; and a coating comprising a bioerodible material or a biodegradable material, wherein the biodegradable material chemically degrades in vivo in a manner that it can release the drug for a period that is significantly greater than the normal in vivo life of the drug. The media composition comprises the drug dispersed in a depot-forming material. The media composition is in a liquid form before administration and is configured to substantially increase in viscosity during or after being injected into a body of a mammal, so that the form of the media after injection is a solid or a gel.

The composite drug delivery material according to the invention is also for use in methods of treating ocular diseases or injuries, wherein the methods comprise injecting a composite drug delivery material as described herein into an eye of a mammal in need thereof.

### DETAILED DESCRIPTION

The composite drug delivery materials described herein comprise a plurality of microparticles dispersed in a media composition. In the composite drug delivery material, the drug is present in both microparticles and the media composition. The media composition comprises a depot-forming material and a drug dispersed in the depot-forming material. The microparticles comprise a drug and other materials such as a bioerodible or a biodegradable coating. Thus, a composite drug delivery material may be an *in vivo, in-situ* drug delivery system. Microparticles comprise coated drug particles or coated drug-polymer particles or microspheres, wherein the coating comprises a bioerodible or biodegradable material.

Before administration, the media is a liquid, such as a low viscosity liquid. Low viscosity may allow the media to be injected. When a media is injected into a body of a mammal, the media substantially increases in viscosity so that the form of the media in a body is a solid or a gel. A substantial increase in viscosity includes any increase in viscosity that would substantially increase the difficulty of injecting a liquid through a needle, such as an increase of about at least about 1000 cP, at least about 10,000 cP, at least about 100,000 cP, at least about 500,000 cP, or at least about 1,000,000 cP. A solid includes a material that has a definite shape. A gel includes a material that has a definite shape under normal conditions, but which may flow upon the application of an external force greater than gravitational force.

A media composition includes any composition comprising a depot-forming material. A depot-forming material includes a material that may be in a liquid form before administration and may substantially increase in viscosity so as to form a solid or a gel during or after being injected into a body of a mammal. Examples of depot forming materials include REGEL®, sucrose acetate isobutyrate complex, poly-lactide-co-glycolide (PLGA) in an organic solution, and polylactide (PLA) in an organic solution.

Some depot-forming materials may comprise PLGA or PLA dissolved in an organic solvent, such as a water-soluble organic solvent. When the media composition is injected, the solvent disperses, leaving a PLGA or PLA depot immersed in an aqueous environment of physiological fluid. Since PLGA or PLA is insoluble in aqueous media, it may quickly precipitate to form a solid on contact with physiological fluid. Examples of suitable organic solvents for a PLGA or PLA media composition may include: N-methyl-2-pyrrolidone, propylene glycol, dimethyl sulfoxide, tetrahydrofuran, triacetin, ethyl benzoate, and benzyl benzoate. In some embodiments, the organic solvent may comprise N-methyl-2-pyrrolidone, benzyl benzoate, or a combination thereof. A PLGA/N-methyl-2-pyrrolidone depot-forming material is available from Atrix Lab under the tradename ELIGARD®. A PLGA/benzyl benzoate depot forming material is available from Alza under the tradename ALZAMER®.

Some depot-forming materials may comprise sucrose acetate isobutyrate dissolved in a water miscible organic solvent such as, ethanol, or benzyl alcohol. The solution has a low viscosity which may improve ease of administration with a small gauge needle. When injected, the sucrose acetate isobutyrate may increase in viscosity to form a gel or a solid. A sucrose acetate isobutyrate depot forming material is available from Durect under the tradename SABER®.

Some depot-forming materials may comprise a thermosensitive biodegradable triblock copolymer comprising hydrophobic PLGA blocks (A) and hydrophilic polyethylene glycol (PEG) blocks (B) with an ABA or BAB block configuration. REGEL®, developed by MacroMed, is an ABA triblock copolymer which is soluble in water. An aqueous solution of REGEL® is a free flowing liquid at 15°C, and transforms into a gel at body temperature when injected. The drug release rate may be adjusted by varying the hydrophilic/hydrophobic content, polymer concentration, molecular weight, and/or polydispersity of the triblock copolymer.

Some depot-forming materials may comprise Poloxamer 407, a triblock copolymer comprising a central hydrophobic block of polypropylene glycol that is flanked by two PEG blocks. It is a water-soluble nonionic surfactant that forms an aqueous solution with reverse-thermal gelation properties. A solution with more than 20% of the polymer exhibits a low viscosity at low temperatures, but rapidly forms a rigid semi-solid gel network at body temperature.

Some depot-forming materials may comprise GELSITE®, available from DelSite Biotech. Inc.). GELSITE® is a natural acidic polysaccharide extracted and purified from an aloe plant. The polymer, in aqueous solution, forms a gel in the presence of calcium when injected, thus entrapping a drug and providing sustained release.

A plurality of microparticles are dispersed in the media composition. The term "dispersed" includes mixing the microparticles in the media composition so that a substantial fraction of the microparticles, such as at least about 50%, at least about 80%, at least about 90%, or at least about 99%, have contact with, or are surrounded by, a media composition. Individual microparticles or clusters of microparticles may be dispersed in a media composition. Microparticles may be dispersed in an approximately homogeneous manner, or may be dispersed heterogeneously.

The microparticles include a drug, including any compound or substance recognized in the official United States Pharmacopoeia, official Homoeopathic Pharmacopoeia of the United States, or official National Formulary, or any supplement to any of them; and any compound or substance intended for use in the diagnosis, cure, mitigation, treatment, or prevention of disease in man or other animals; and any substance other than food or water intended to affect the structure or any function of the body of man or other animals.

Some examples of drugs may include: ace-inhibitors, endogenous cytokines, agents that influence basement membrane, agents that influence the growth of endothelial cells, adrenergic agonists or blockers, cholinergic agonists or blockers, aldose reductase inhibitors, analgesics, anesthetics, antiallergics, anti-inflammatory agents, antihypertensives, pressors, antibacterials, antivirals, antifungals, antiprotozoals, anti-infectives, antitumor agents, antimetabolites, antiangiogenic agents, tyrosine kinase inhibitors, antibiotics such as aminoglycosides such as gentamycin, kanamycin, neomycin, and vancomycin; amphenicols such as chloramphenicol; cephalosporins, such as cefazolin HCI; penicillins such as ampicillin, penicillin, carbenicillin, oxycillin, methicillin; lincosamides such as lincomycin; polypeptide antibiotics such as polymixin and bacitracin; tetracyclines such as tetracycline; quinolones such as ciproflaxin; sulfonamides such as chloramine T; and sulfones such as sulfanilic acid as the hydrophilic entity, anti-viral drugs, e.g. acyclovir, gancyclovir, vidarabine, azidothymidine, dideoxyinosine, dideoxycytosine, dexamethasone , ciproflaxin, water soluble antibiotics, such as acyclovir, gancyclovir, vidarabine, azidothymidine, dideoxyinosine, dideoxycytosine; epinephrine; isoflurphate; adriamycin; bleomycin; mitomycin; ara-C; actinomycin D; and scopolamine; analgesics, such as codeine, morphine, ketorolac, naproxen, an anesthetic, e.g. lidocaine; adrenergic agents such as α-adrenergic blockers, β-adrenergic blockers, α-adrenergic agonists such as alpha-2 adrenergic receptor agonists, β-adrenergic agonists, e.g. ephidrine, epinephrine, timolol, and brimonidine; aldose reductase inhibitor, e.g. epalrestat, ponalrestat, sorbinil, tolrestat; antiallergic, e.g. cromolyn, beclomethasone, dexamethasone, and flunisolide; colchicine, anihelminthic agents, e.g. ivermectin and suramin sodium; antiamebic agents, e.g. chloroquine and chlortetracycline; and antifungal agents, e.g. amphotericin, anti-angiogenesis compounds such as anecortave acetate, retinoids such as Tazarotene, anti-glaucoma agents, such as brimonidine (ALPHAGAN® and ALPHAGAN P®), acetozolamide, bimatoprost (LUMIGAN®), Timolol, mebefunolol; memantine;; 2ME2; anti-neoplastics, such as vinblastine, vincristine, interferons; α, β and y, antimetabolites, such as folic acid analogs, purine analogs, and pyrimidine analogs; immunosuppressants such as azathiprine, cyclosporine and mizoribine; miotic agents, such as carbachol, mydriatic agents such as atropine, ranibizumab, bevacizumab, protease inhibitors such as aprotinin, camostat, gabexate, vasodilators such as bradykinin, and various growth factors, such epidermal growth factor, basic fibroblast growth factor, and nerve growth factors. In some embodiments the drug is timolol, brimonidine, bimatoprost, ketorolac, dexamethasone, memantine, prednisolone acetate, triamcinolone acetonide, ranibizumab, or bevacizumab. Reference to a drug includes a pharmaceutically acceptable salt or a prodrug of the drug.

In some embodiments, two or more different drugs may be used in a formulation. The drugs may be mixed together in any of the microspheres or may be mixed together in the media composition, or may be separated. For example, one set of microspheres may contain one drug and a different set of microspheres may contain a second drug. In some embodiments, a formulation comprising a first set of microspheres comprising ranibizumab or bevacizumab and a second set of microspheres comprising a different drug may be used to treat macular degeneration or diabetic retinopathy.

After the media material increases in viscosity to form a solid or a gel, microparticles and drug not part of a microparticle ("media drug") may be encapsulated in the solid or gel so that contact between physiological fluid and media drug may be limited. Over time, aqueous physiological fluid may slowly permeate the media material and release the media drug. Eventually, physiological fluid may also begin penetrating the microparticles so that drug may begin to be released from the microparticles. Media drug composition may be released up to 1 month, up to 2 months, up to 3 months, or possibly longer, after injection.

A liquid media composition, such as a low viscosity liquid media composition, may allow a drug to be delivered through a very small needle, such as about 25 gauge or about 30 gauge. Use of a small needle may reduce the risk of needle-induced serious adverse effects. Use of a small needle may be especially beneficial for injection of a formulation comprising a drug into the vitreous humor of an eye. For injection into a back of an eye area such as a vitreous humor, a smaller needle reduces the likelihood of needle-induced serious adverse effects, such as endophthalmitis, retinal separation, vitreal separation, and severe pain.

Injecting a low viscosity liquid media composition that solidifies or gels *in vivo* provides significant safety advantages over other methods of implanting solid devices. For example, injecting a sustained release liquid to solid formulation may avoid surgical implantation of a solid device of a similar size into an eye. A narrow gauge needle may minimize trauma to an eye as compared to surgical implantation, and thus may minimize loss of vitreal fluid, may decrease the probability of retinal and vitreal separation, and may greatly reduce the risk of infection such as endophthalmitis.

A solidifying or gelling media may trap microparticles and drug particles as the formulation enters the vitreous. Liquid formulations comprising microparticles or drug particles that do not undergo a phase change may "plume" and/or settle on the posterior retina, which may cause temporary blindness. Furthermore, formulations that plume may settle on the lens, where they may cause vitreous opacities or speckling. Small particles may also be endocytosed within a cell, where they are rapidly degraded and "dumped," thereby initiating a local inflammatory response.

Penetration of microparticles may begin after a substantial amount of drug has been released from the media. If the thickness of microparticle coatings varies within a plurality of microparticles, particles with thinner coatings may begin releasing drug before particles with thicker coatings. Thus, a range of coating thickness may help to control drug release over time. A plurality of microspheres may have a variety of coating materials, such that different coating materials allow different release properties. Thus, a variety of coating materials may help to control drug release over time.

Microparticles include any particles having a size around the micron or µm range, or smaller, such as about 0.01 µm to about 1000 µm, about 1 µm to about 300 µm, about 10 µm to about 100 µm. Microparticles may be of any shape, such as approximately spherical (e.g. microspheres), spheroid, ellipsoid, cylindrical, or rod-shaped.

Since microparticles are very small, they may be delivered through a very small needle, such as about 25 gauge or about 30 gauge. Use of a small needle may reduce the risk of needle-induced serious adverse effects, as explained above.

A microparticle comprises a drug, as described above, and a coating. The coating comprises a bioerodible material or a biodegradable material.

A bioerodible material includes any material that erodes *in vivo.* Bioerodible materials do not necessarily chemically degrade *in vivo,* but may disperse or dissolve *in vivo* in a manner that can release a therapeutically effective amount of a drug for a period that is significantly greater than the normal *in vivo* life of the drug. Examples of bioerodible materials include: polyvinylpyrrolidine (PVP), carboxymethylcellulose (CMC), polyvinyl chloride (PVC), hydroxypropylmethylcellulose (HPMC), and polyorthoester.

A biodegradable material includes a material which chemically degrades *in vivo* in a manner that it can release a therapeutically effective amount of drug for a period that is significantly greater than the normal *in vivo* life of the drug. A biodegradable material may be a single polymer or copolymer, or any combination or blend of polymers. Examples of biodegradable polymer materials may include: polymers made of monomers such as esters, ethers, anhydrides, amides, orthoesters, which when degraded result in physiologically acceptable degradation products. Polymer materials may be crosslinked or non-crosslinked. If crosslinked, they may be lightly crosslinked, such as less than 5% or 1% crosslinked.

Some biodegradable polymer materials may include polymers of hydroxyaliphatic carboxylic acids, either homo- or copolymers, and polysaccharides. Included among polyesters of interest are homo- or copolymers of D-lactic acid, L-lactic acid, racemic lactic acid, glycolic acid, caprolactone, and combinations thereof. For some copolymers of glycolic and lactic acid, biodegradation may be affected by the ratio of glycolic to lactic acid.

In some embodiments, biodegradable polymer material may comprise PLA, PLGA, a polyanhydride, or a polyorthosters (POE), or a combination thereof. In some embodiments, polylactide may be about 2% to about 100 %, about 2% to about 20%, about 20% to about 30%, about 30% to about 40%, about 40% to about 50%, about 50% to about 60%, about 60% to about 70%, about 70% to about 80%, about 80% to about 90%, or about 90% to about 100% of the weight of the polymer material. In some embodiments, poly(lactide-co-glycolide) may be about 2% to about 100%, about 2% to about 20%, about 20% to about 30%, about 30% to about 40%, about 40% to about 50%, about 50% to about 60%, about 60% to about 70%, about 70% to about 80%, about 80% to about 90%, or about 90% to about 100% of the weight of the polymer material.

Polylactide, polylactic acid, or PLA, includes poly (D,L-lactide), and may also be identified by CAS Number 26680-10-4 and may be represented by a formula:

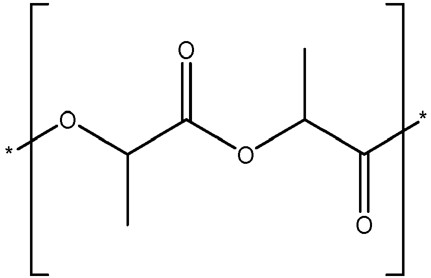

Poly(lactic-co-glycolic)acid, poly(lactide-co-glycolide), or PLGA, includes (D,L-lactide-co-glycolide), also identified by CAS Number 26780-50-7, and may be represented by a formula:

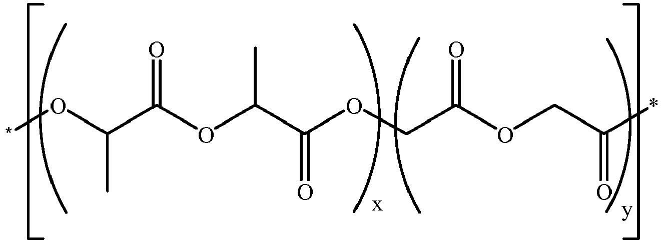

Thus, PLGA comprises one or more blocks of D,L-lactide repeat units and one or more blocks of glycolide repeat units, where the size and number of the respective blocks may vary.

The molar percent of each monomer in poly(lactic-co-glycolic)acid (PLGA) copolymer may be 0-100%, about 15-85%, about 25-75%, or about 35-65%. In some embodiments, the D,L-lactide may be about 50% to about 75%, about 48% to about 52%, or about 50%; or about 73% to about 77%, or about 75% of the PLGA polymer on a molar basis. The balance of the polymer may essentially be glycolide repeat units. For example, glycolide may be about 25% to about 50%, about 23% to about 27%, or about 25%; or about 48% to about 52%, or about 50% of the PLGA polymer on a molar basis. Other groups, such as terminal or capping groups may be present in small amounts. In some embodiments, PLGA copolymers are used in conjunction with polylactide polymers.

In some embodiments, a coating may comprise PLA, PLGA, PVP, CMC, PVC, HPMC, polyorthoester, or PEG.

In some embodiments, a plurality of microspheres have 3 different coatings, a first group having a PLGA coating, a second group having a PLA coating, and a third group having a POE coating.

The thickness of a coating may vary. In some embodiments, a coating may have a thickness of about 1 µm to about 5 µm, about 1µm, about 2 µm, about 3 µm, about 4 µm, about 5 µm, or any thickness in a range bounded by, or between, any of these values.

In some embodiments, a plurality of microparticles may have several different coating thicknesses. For example, microparticles may have 2, 3, 4, 5, or more different coating thicknesses, and or types of coating materials. In some embodiments, the microparticles may have:
a) 2 coating thicknesses such as: about 1 µm and about 2 µm, 1 µm and 3 µm, 1 µm and 4 µm, 1 µm and 5 µm, 2 µm and 3 µm, 2 µm and 4 µm, 2 µm and 5 µm, 3 µm and 4 µm, 3 µm and 5 µm, 4 µm and 5 µm;
b) 3 coating thicknesses such as 1 µm, 2 µm, and 3 µm; 1 µm, 2 µm, and 4 µm; 1 µm, 3 µm, and 4 µm; 2 µm, 3 µm, and 4 µm; 1 µm, 2 µm, and 5 µm; 1 µm, 3 µm, and 5 µm; 2 µm, 3 µm, and 5 µm; 1 µm, 4 µm, and 5 µm; 2 µm, 4 µm, and 5 µm; 3 µm, 4 µm, and 5 µm; 1 µm, 2 µm, 3 µm, 4 µm, and 5 µm;
c) 4 coating thicknesses such as 2 µm, 3 µm, 4 µm, and 5 µm; 1 µm, 3 µm, 4 µm, and 5 µm; 1 µm, 2 µm, 4 µm, and 5 µm; 1 µm, 2 µm, 3 µm, and 5 µm; 1 µm, 2 µm, 3 µm, and 4 µm; or
d) 5 coating thicknesses such as 1 µm, 2 µm, 3 µm, 4 µm, and 5 µm.

In some embodiments, a plurality of microspheres may comprise a first microsphere type comprising a PLA coating with a thickness of about 1 µm; a second microsphere type comprising a PLA coating with a thickness of about 2 µm; a third microsphere type comprising a PLA coating with a thickness of about 3 µm; a fourth microsphere type comprising a PLA coating with a thickness of about 4 µm; and a fifth microsphere type comprising a PLA coating with a thickness of about 5 µm.

Some examples of about 1 µm may include: 0.5 µm, 0.6 µm, 0.7 µm, 0.8 µm, 0.9 µm, 1.0 µm, 1.1 µm, 1.2 µm, 1.3 µm, 1.4 µm.

Some examples of about 2 µm may include: 1.5 µm, 1.6 µm, 1.7 µm, 1.8 µm, 1.9 µm, 2.0 µm, 2.1 µm, 2.2 µm, 2.3 µm, 2.4 µm.

Some examples of about 3 µm may include: 2.5 µm, 2.6 µm, 2.8 µm, 2.8 µm, 2.9 µm, 3.0 µm, 3.1 µm, 3.2 µm, 3.3 µm, 3.4 µm.

Some examples of about 4 µm may include: 3.5 µm, 3.6 µm, 3.7 µm, 3.8 µm, 3.9 µm, 4.0 µm, 4.1 µm, 4.2 µm, 4.3 µm, 4.4 µm.

Some examples of about 5 µm may include: 4.5 µm, 4.6 µm, 4.7 µm, 4.8 µm, 4.9 µm, 5.0 µm, 5.1 µm, 5.2 µm, 5.3 µm, 5.4 µm.

In addition to being coated with a bioerodible material or biodegradable material, a microparticle may include a bioerodible material or a biodegradable material inside the coating with the drug, and may thus further extend the delivery of a drug. If the interior of a microparticle comprises a bioerodible material or a biodegradable material, the amount may vary. In some embodiments, bioerodible material or biodegradable material may be about 10% to about 90%, about 10% to about 30%, about 30% to about 70%, about 30% to about 40%, about 40% to about 50%, about 50% to about 60%, about 70% to about 90%, or about 50% to about 85% of a microparticle by weight.

The amount of drug in a microparticle may vary. In some embodiments, drug may be about 10% to about 90%, about 10% to about 30%, about 30% to about 70%, about 30% to about 40%, about 40% to about 50%, about 50% to about 60%, or about 70% to about 90% of a microparticle by weight.

In some embodiments, microparticles may comprise PLGA, PLA, or a combination thereof loaded with therapeutically effective agent and a biodegradable or bioerodible coating.

In some embodiments, a composite drug delivery material may deliver an effective amount of the drug at the site of injection for a longer period of time than: a composition comprising the media material and the same amount of the drug without any microparticles and/or a plurality of microparticles comprising the same amount of the drug without any media material.

In some embodiments, a composite drug delivery material may provide sustained release of a drug over a period of about 1 month to about 3 years, about 2 month to about 2 years, about 3 months to about 12 months, at least about 2 months, about 3 months, about 6 months, about 12 months, or more. This sustained release may reduce the risk of needle-induced serious adverse events because it may reduce the frequency of injections. Reducing the frequency of injections into an eye may reduce the risk of needle-induced serious adverse events, and may be preferred by patients. Thus, a drug delivery system that allows injections to be at least about 3 months apart, about 6 months apart, about 9 months apart, about 12 months apart, or longer, may be desirable.

In some embodiments, pseudo-zero order delivery of a drug may be obtainable over an extended period of time by varying (1) coating thickness of drug-loaded microparticles, (2) the total number of different thicknesses of coatings on drug-loaded microparticles (3), the number of microparticles within each coating group, and/or (4) the biodegradable or bioerodible polymer used to prepare the microparticle.

The composite drug delivery material is for use in treating any ocular disease or injury, including the following:

MACULOPATHIES/RETINAL DEGENERATION: Non-Exudative Age Related Macular Degeneration (ARMD), Exudative Age Related Macular Degeneration (ARMD), Choroidal Neovascularization, Diabetic Retinopathy, Acute Macular Neuroretinopathy, Central Serous Chorioretinopathy, Cystoid Macular Edema, Diabetic Macular Edema.

UVEITIS/RETINITIS/CHOROIDITIS: Acute Multifocal Placoid Pigment Epitheliopathy, Behcet's Disease, Birdshot Retinochoroidopathy, Infectious (Syphilis, Lyme, Tuberculosis, Toxoplasmosis), Intermediate Uveitis (Pars Planitis), Multifocal Choroiditis, Multiple Evanescent White Dot Syndrome (MEWDS), Ocular Sarcoidosis, Posterior Scleritis, Serpignous Choroiditis, Subretinal Fibrosis and Uveitis Syndrome, Vogt-Koyanagi-Harada Syndrome.

VASUCLAR DISEASES/ EXUDATIVE DISEASES Retinal Arterial Occlusive Disease, Central Retinal Vein Occlusion, Disseminated Intravascular Coagulopathy, Branch Retinal Vein Occlusion, Hypertensive Fundus Changes, Ocular Ischemic Syndrome, Retinal Arterial Microaneurysms, Coat's Disease, Parafoveal Telangiectasis, Hemi-Retinal Vein Occlusion, Papillophlebitis, Central Retinal Artery Occlusion, Branch Retinal Artery Occlusion, Carotid Artery Disease (CAD), Frosted Branch Angiitis, Sickle Cell Retinopathy and other Hemoglobinopathies, Angioid Streaks, Familial Exudative Vitreoretinopathy, Eales Disease.

TRAUMATIC/SURGICAL: Sympathetic Ophthalmia, Uveitic Retinal Disease, Retinal Detachment, Trauma, Laser, PDT, Photocoagulation, Hypoperfusion During Surgery, Radiation Retinopathy, Bone Marrow Transplant Retinopathy.

PROLIFERATIVE DISORDERS: Proliferative Vitreal Retinopathy and Epiretinal Membranes, Proliferative Diabetic Retinopathy, Retinopathy of Prematurity (retrolental fibroplastic).

INFECTIOUS DISORDERS: Ocular Histoplasmosis, Ocular Toxocariasis, Presumed Ocular Histoplasmosis Syndrome (POHS), Endophthalmitis, Toxoplasmosis, Retinal Diseases Associated with HIV Infection, Choroidal Disease Associated with HIV Infection, Uveitic Disease Associated with HIV Infection, Viral Retinitis, Acute Retinal Necrosis, Progressive Outer Retinal Necrosis, Fungal Retinal Diseases, Ocular Syphilis, Ocular Tuberculosis, Diffuse Unilateral Subacute Neuroretinitis, Myiasis.

GENETIC DISORDERS: Retinitis Pigmentosa, Systemic Disorders with Associated Retinal Dystrophies, Congenital Stationary Night Blindness, Cone Dystrophies, Fundus Flavimaculatus, Best's Disease, Pattern Dystrophy of the Retinal Pigmented Epithelium, X-Linked Retinoschisis, Sorsby's Fundus Dystrophy, Benign Concentric Maculopathy, Bietti's Crystalline Dystrophy, pseudoxanthoma elasticum, Osler Weber syndrome.

RETINAL TEARS/HOLES: Retinal Detachment, Macular Hole, Giant Retinal Tear.

TUMORS: Retinal Disease Associated with Tumors, Solid Tumors, Tumor Metastasis, Benign Tumors, for example, hemangiomas, neurofibromas, trachomas, and pyogenic granulomas, Congenital Hypertrophy of the RPE, Posterior Uveal Melanoma, Choroidal Hemangioma, Choroidal Osteoma, Choroidal Metastasis, Combined Hamartoma of the Retina and Retinal Pigmented Epithelium, Retinoblastoma, Vasoproliferative Tumors of the Ocular Fundus, Retinal Astrocytoma, Intraocular Lymphoid Tumors.

MISCELLANEOUS: Punctate Inner Choroidopathy, Acute Posterior Multifocal Placoid Pigment Epitheliopathy, Myopic Retinal Degeneration, Acute Retinal Pigment Epithelitis, Ocular inflammatory and immune disorders, ocular vascular malfunctions, Corneal Graft Rejection, and Neovascular Glaucoma.

### Example 1

A batch of drug-loaded microspheres is separated into 3 portions. The first portion is coated with PLGA. The second portion is coated with PLA having a second thickness. The third portion is coated with a polyorthoester [POE] having a third thickness. The POE is a poly(orthoester) polymer made by condensation polymerization of 3,9-diethylidene-2,4,8,10-tetraoxaspiro[5.5]undecane (DETOSU), cyclohexanedimethanol (CDM), triethylene glycol (TEG) and 1,10-decanediol (DD). Triethylene glycol glycolide (TEG-GL) is added as a latent acid catalyst to initiate hydrolysis of the POE backbone. The POE polymer molecular weight is about 30,000 to about 35,000 Daltons. Coated microspheres from the three groups are mixed with a solidifying depot-forming media containing the same drug. Once the suspension is injected into the vitreous through a narrow gauge needle, it solidifies nearly instantly, trapping the microspheres. As the depot slowly erodes, the drug from the depot is released first. This is followed by release from the microspheres in the first group, the second group, and the third group, respectively.

### Example 2

PLGA microspheres loaded with Compound 1 with PLA coatings of 1, 2, 3, 4, and 5 µm thickness respectively, are suspended in a formulation of Compound 1 in ReGel and adjusted for viscosity, pH, and ionic strength. The suspension of microspheres is then lyophilized for stability purposes and stored (the microspheres may also be lyophilized in the presence of "free" drug, so as to provide a bolus dose, if desirable, upon reconstitution and injection). After reconstitution, the formulation, maintained at 5°C, is injected through a 30 gauge needle into an animal vitreous and the formulation instantly gels at the temperature of the vitreous, ca 37°C, trapping the microspheres.

Once in the vitreous, the ReGel begins to release Compound 1. As the ReGel bioerodes, additional drug is released and the coated microspheres begin to be exposed to the aqueous environment within the vitreous. As the microspheres are exposed to the aqueous environment at 37°C, the coatings begin to bioerode.

The microspheres within the 1 µm coating have its drug-containing PLGA matrix exposed first and begin to release Compound 1. Meanwhile, microspheres with intact coating do not yet release the drug. As time continues, the coating on the microspheres within the 2 µm coating erode and begin to expose its drug-containing PLGA matrix, and drug begins to be released. Around the same time, the drug in the ReGel and the microspheres in the 1 µm coating begins to be depleted.

Sometime later, the 3 µm coating on a third group of microspheres is bio-removed and this group begins releasing Compound 1, as the drug release from the earlier releasing microspheres (e.g. those with 1 µm and 2 µm coatings) is depleted. This is followed, sometime later, by the erosion of the 4 µm and 5 µm coatings from the heaviest-coated microspheres, and subsequently, further drug is released.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained. At the very least, and not as an attempt to limit the application of the doctrine of equivalents to the scope of the claims, each numerical parameter should at least be construed in light of the number of reported significant digits and by applying ordinary rounding techniques.

The terms "a," "an," "the" and similar referents used in the context of describing the invention (especially in the context of the following claims) are to be construed to cover both the singular and the plural, unless otherwise indicated herein or clearly contradicted by context. All methods described herein can be performed in any suitable order unless otherwise indicated herein or otherwise clearly contradicted by context.

Certain embodiments are described herein, including the best mode known to the inventors for carrying out the invention.

In closing, it is to be understood that the embodiments disclosed herein are illustrative of the principles of the claims.

## Claims

1. A composite drug delivery material comprising:
a plurality of microparticles dispersed in a media composition;
wherein the microparticles comprise:
a drug; and
a coating comprising a bioerodible material or a biodegradable material, wherein the biodegradable material chemically degrades in vivo in a manner that it can release the drug for a period that is significantly greater than the normal in vivo life of the drug; and
wherein the media composition comprises the drug dispersed in a depot-forming material;
and wherein the media composition is in a liquid form before administration and is configured to substantially increase in viscosity during or after being injected into a body of a mammal, so that the form of the media after injection is a solid or a gel.

2. The composite drug delivery material according to claim 1 for use in a method of treating an ocular disease or injury, wherein the method comprises injecting the composite drug delivery material into an eye of a mammal in need thereof.

3. The composite drug delivery material for use according to claim 2, wherein the ocular disease is macular degeneration or diabetic retinopathy.

4. The composite drug delivery material or composite drug delivery material for use according to any one of the preceding claims, wherein the drug is timolol, brimonidine, bimatoprost, ketorolac, dexamethasone, memantine, prednisolone acetate, triamcinolone acetonide, ranibizumab, or bevacizumab.

5. The composite drug delivery material or composite drug delivery material for use according to any one of the preceding claims, wherein the drug is ranibizumab.

6. The composite drug delivery material or composite drug delivery material for use according to any one of the preceding claims, wherein the coating comprises polylactide, poly-lactide-co-glycolide, polyvinylpyrrolidine, carboxymethylcellulose, PVC, hydroxymethylpropylcellulose, polyorthoester, or a polyethylene glycol.

7. The composite drug delivery material or composite drug delivery material for use according to any one of the preceding claims, wherein the coating comprises polylactide.

8. The composite drug delivery material or composite drug delivery material for use according to any one of the preceding claims, wherein the coating has a thickness of about 1 µm to about 5 µm.

9. The composite drug delivery material or composite drug delivery material for use according to any one of the preceding claims, wherein the plurality of microparticles comprises:
a first microsphere type comprising a polymer coating having a thickness of about 1 µm;
a second microsphere type comprising a polymer coating having a thickness of about 2 µm;
a third microsphere type comprising a polymer coating having a thickness of about 3 µm;
a fourth microsphere type comprising a polymer coating having a thickness of about 4 µm; and
a fifth microsphere type comprising a polymer coating having a thickness of about 5 µm.

10. The composite drug delivery material or composite drug delivery material for use according to any one of the preceding claims, wherein the microspheres further comprise poly-lactide-co-glycolide, polylactide, or a combination thereof loaded with drug.

11. The composite drug delivery material or composite drug delivery material for use according to any one of the preceding claims, wherein the depot-forming material comprises: a biodegradable copolymer comprising poly-lactide-co-glycolide blocks and polyethylene glycol blocks, a sucrose acetate isobutyrate complex, a poly-lactide-co-glycolide in an organic solution, or a polylactide in an organic solution.

12. The composite drug delivery material or composite drug delivery material for use according to any one of the preceding claims, wherein the polymer is a polylactide polymer.

## Patentansprüche

1. Arzneimittelabgabe-Kompositmaterial, umfassend:
eine Vielzahl von Mikropartikeln, die in einer Medienzusammensetzung dispergiert sind,
wobei die Mikropartikel umfassen:
ein Arzneimittel und
eine Beschichtung, umfassend ein bioerodierbares Material oder ein biologisch abbaubares Material, wobei sich das biologisch abbaubare Material in vivo in einer Weise abbaut, dass es das Arzneimittel über eine Dauer freisetzt, die signifikant länger als die normale in vivo-Lebensdauer des Arzneimittels ist, und wobei die Medienzusammensetzung das in einem Depot-bildenden Material dispergierte Arzneimittel umfasst,
und wobei die Medienzusammensetzung vor Verabreichung in flüssiger Form vorliegt und so konfiguriert ist, dass ihre Viskosität, während oder nachdem sie in einen Körper eines Säugetieres injiziert wurde, wesentlich zunimmt, so dass die Form des Mediums nach Injektion ein Feststoff oder ein Gel ist.

2. Arzneimittelabgabe-Kompositmaterial gemäß Anspruch 1 zur Verwendung in einem Verfahren zur Behandlung einer Augenerkrankung oder -verletzung, wobei das Verfahren das Injizieren des Arzneimittelabgabe-Kompositmaterials in ein Auge eines Säugetiers mit Bedarf daran umfasst.

3. Arzneimittelabgabe-Kompositmaterial zur Verwendung gemäß Anspruch 2, wobei die Augenerkrankung eine Makuladegeneration oder diabetische Retinopathie ist.

4. Arzneimittelabgabe-Kompositmaterial oder Arzneimittelabgabe-Kompositmaterial zur Verwendung gemäß irgendeinem der vorhergehenden Ansprüche, wobei das Arzneimittel Timolol, Brimonidin, Bimatoprost, Ketorolac, Dexamethason, Memantin, Prednisolonacetat, Triamcinolonacetonid, Ranibizumab oder Bevacizumab ist.

5. Arzneimittelabgabe-Kompositmaterial oder Arzneimittelabgabe-Kompositmaterial zur Verwendung gemäß irgendeinem der vorhergehenden Ansprüche, wobei das Arzneimittel Ranibizumab ist.

6. Arzneimittelabgabe-Kompositmaterial oder Arzneimittelabgabe-Kompositmaterial zur Verwendung gemäß irgendeinem der vorhergehenden Ansprüche, wobei die Beschichtung Polylactid, Polylactid-co-Glycolid, Polyvinylpyrrolidon, Carboxymethylcellulose, PVC, Hydroxymethylpropylcellulose, Polyorthoester oder ein Polyethylenglycol umfasst.

7. Arzneimittelabgabe-Kompositmaterial oder Arzneimittelabgabe-Kompositmaterial zur Verwendung gemäß irgendeinem der vorhergehenden Ansprüche, wobei die Beschichtung Polylactid umfasst.

8. Arzneimittelabgabe-Kompositmaterial oder Arzneimittelabgabe-Kompositmaterial zur Verwendung gemäß irgendeinem der vorhergehenden Ansprüche, wobei die Beschichtung eine Dicke von etwa 1 µm bis etwa 5 µm aufweist.

9. Arzneimittelabgabe-Kompositmaterial oder Arzneimittelabgabe-Kompositmaterial zur Verwendung gemäß irgendeinem der vorhergehenden Ansprüche, wobei die Vielzahl an Mikropartikeln umfasst:
einen ersten Mikrosphärentyp, umfassend eine Polymerbeschichtung mit einer Dicke von etwa 1 µm,
einen zweiten Mikrosphärentyp, umfassend eine Polymerbeschichtung mit einer Dicke von etwa 2 µm,
einen dritten Mikrosphärentyp, umfassend eine Polymerbeschichtung mit einer Dicke von etwa 3 µm,
einen vierten Mikrosphärentyp, umfassend eine Polymerbeschichtung mit einer Dicke von etwa 4 µm und
einen fünften Mikrosphärentyp, umfassend eine Polymerbeschichtung mit einer Dicke von etwa 5 µm.

10. Arzneimittelabgabe-Kompositmaterial oder Arzneimittelabgabe-Kompositmaterial zur Verwendung gemäß irgendeinem der vorhergehenden Ansprüche, wobei die Mikrosphären ferner mit dem Arzneimittel beladenes Polylactid-co-Glycolid, Polylactid oder eine Kombination davon umfassen.

11. Arzneimittelabgabe-Kompositmaterial oder Arzneimittelabgabe-Kompositmaterial zur Verwendung gemäß irgendeinem der vorhergehenden Ansprüche, wobei das Depot-bildende Material umfasst: ein biologisch abbaubares Copolymer, umfassend Polylactid-co-Glycolid-Blöcke und Polyethylenglycol-Blöcke, einen Sucroseacetatisobutyrat-Komplex, ein Polylactid-co-Glycolid in einer organischen Lösung oder ein Polylactid in einer organischen Lösung.

12. Arzneimittelabgabe-Kompositmaterial oder Arzneimittelabgabe-Kompositmaterial zur Verwendung gemäß irgendeinem der vorhergehenden Ansprüche, wobei das Polymer ein Polylactid-Polymer ist.

## Revendications

1. Matériau d'administration de médicament composite comprenant :
une pluralité de microparticules dispersées dans une composition de milieu ;
dans lequel les microparticules comprennent :
un médicament ; et
un revêtement comprenant un matériau bioérodable ou un matériau biodégradable, dans lequel le matériau biodégradable se dégrade chimiquement in vivo d'une manière qui peut libérer le médicament pendant une période qui est significativement supérieure à la vie in vivo normale du médicament ; et
dans lequel la composition de milieu comprend le médicament dispersé dans un matériau de formation de dépôt ;
et dans lequel la composition de milieu se trouve sous une forme liquide avant l'administration et est configurée pour que augmenter sensiblement en viscosité durant ou après avoir été injectée dans un corps d'un mammifère, de manière à ce que la forme du milieu après l'injection soit un solide ou un gel.

2. Matériau d'administration de médicament composite selon la revendication 1 pour une utilisation dans un procédé de traitement d'une maladie ou d'une lésion oculaire, dans lequel le procédé comprend l'injection du matériau d'administration de médicament composite dans un œil d'un mammifère nécessitant celui-ci.

3. Matériau d'administration de médicament composite pour une utilisation selon la revendication 2, dans lequel la maladie oculaire est la dégénérescence maculaire ou la rétinopathie diabétique.

4. Matériau d'administration de médicament composite ou matériau d'administration de médicament composite pour une utilisation selon l'une quelconque des revendications précédentes, dans lequel le médicament est le timolol, la brimonidine, le bimatoprost, le kétorolac, la dexaméthasone, la mémantine, l'acétate de prédnisolone, l'acétonide de triamcinolone, le ranibizumab, ou le bévacizumab.

5. Matériau d'administration de médicament composite ou matériau d'administration de médicament composite pour une utilisation selon l'une quelconque des revendications précédentes, dans lequel le médicament est le ranibizumab.

6. Matériau d'administration de médicament composite ou matériau d'administration de médicament composite pour une utilisation selon l'une quelconque des revendications précédentes, dans lequel le revêtement comprend du polylactide, du polylactide-co-glycolide, de la polyvinylpyrrolidine, de la carboxyméthylcellulose, du PVC, de l'hydroxyméthylpropylcellulose, un polyorthoester, ou un polyéthylène glycol.

7. Matériau d'administration de médicament composite ou matériau d'administration de médicament composite pour une utilisation selon l'une quelconque des revendications précédentes, dans lequel le revêtement comprend du polylactide.

8. Matériau d'administration de médicament composite ou matériau d'administration de médicament composite pour une utilisation selon l'une quelconque des revendications précédentes, dans lequel le revêtement présente une épaisseur d'environ 1 µm à environ 5 µm.

9. Matériau d'administration de médicament composite ou matériau d'administration de médicament composite pour une utilisation selon l'une quelconque des revendications précédentes, dans lequel la pluralité de microparticules comprend :
un premier type de microsphères comprenant un revêtement polymère présentant une épaisseur d'environ 1 µm ;
un deuxième type de microsphères comprenant un revêtement polymère présentant une épaisseur d'environ 2 µm ;
un troisième type de microsphères comprenant un revêtement polymère présentant une épaisseur d'environ 3 µm ;
un quatrième type de microsphères comprenant un revêtement polymère présentant une épaisseur d'environ 4 µm ; et
un cinquième type de microsphères comprenant un revêtement polymère présentant une épaisseur d'environ 5 µm.

10. Matériau d'administration de médicament composite ou matériau d'administration de médicament composite pour une utilisation selon l'une quelconque des revendications précédentes, dans lequel les microsphères comprennent en outre du poly-lactide-co-glycolide, du polylactide, ou une combinaison de ceux-ci chargée de médicament.

11. Matériau d'administration de médicament composite ou matériau d'administration de médicament composite pour une utilisation selon l'une quelconque des revendications précédentes, dans lequel le matériau formant dépôt comprend : un copolymère biodégradable comprenant des séquences de poly-lactide-co-glycolide et des séquences de polyéthylène glycol, un complexe d'acétate isobutyrate de saccharose, un poly-lactide-co-glycolide dans une solution organique, ou un polylactide dans une solution organique.

12. Matériau d'administration de médicament composite ou matériau d'administration de médicament composite pour une utilisation selon l'une quelconque des revendications précédentes, dans lequel le polymère est un polymère de polylactide.
